# EUROPEAN PATENT SPECIFICATION

(11) **EP 1 131 765 B1**
(45) Date of publication and mention of the grant of the patent: **24.01.2007**
(21) Application number: 99946567.7
(22) Date of filing: 21.08.1999
(51) Int. Cl.: G06F 19/00

(54) **DNA MARKER PROFILE DATA ANALYSIS**
DATENANALYSE VON DNS-MARKIERUNGSPROFILEN
ANALYSE DE DONNEES DE PROFIL DE MARQUEUR D'ADN

(43) Date of publication of application: 12.09.2001
(73) Proprietor: Agency for Science, Technology and Research, Singapore 138668 (SG)
(72) Inventor: HONG, Yan, Singapore 650206 (SG); CHUAH, Aaron, Singapore 609776 (SG)
(74) Representative: Marchant, James Ian
(86) International application number: PCT/SG1999/000087
(87) International publication number: WO 2001/015057

(56) References cited:
- EP-A- 0 497 468
- WO-A-97/27317
- WO-A-98/40395
- US-A- 5 754 524

## Description

### BACKGROUND OF THE INVENTION

### Field of the Invention

The present invention relates generally to DNA marker analysis and more particularly to methods for processing raw DNA marker profile data into a format that facilitates analysis of the raw data.

### Background and Related Art

DNA markers are molecular genetic markers identified by studying genomic DNA samples. The genome of higher organisms is contained in rod-like structures of tightly coiled DNA (deoxyribonucleic acid) found in the cell nucleus of plants and animals, called chromosomes. Every strand of DNA has pieces or sequences of nucleotides or bases (consisting of adenine, guanine, cytosine and thymine) that contain genetic information which contribute to the function of a gene (exons) and sequences that apparently supply no relevant genetic information at all (introns, minisatellites or microsatellites). Intron, minisatellite or microsatellite sequences are repeated within the gene and in other genes of a DNA sample. Each organism has a unique pattern of these minisatellites (the only exception being multiple individuals from a single zygote, i.e., identical twins), which can be analyzed.

DNA markers are used for mapping and tagging physical traits of interest, and as indicators of genetic diversity. DNA polymorphism can be used for investigating the organization of genomes and for the construction of genetic maps, which can provide detailed blueprints for strategies of gene isolation by map-based cloning, marker-assisted selection, and introgression and dissection of complex traits. Genetic mapping offers modern breeders and scientists a powerful array of tools for analyzing the inheritance of important physiological traits in animals and plants.

The investigation of taxonomic units at the species level and the determination of the uniqueness of a species are essential for conservational, systemic, ecological and evolutionary studies. Knowledge of genetic relationships among genotypes in plant breeding programs permits the organization of germplasm, including elite lines, and provides for more efficient parental selection. Exotic germplasms are important sources of genes exerting highly desirable qualitative effects on traits, such as biotic and abiotic stress resistance. Transfer of such genes in breeding programs can be enhanced through marker-assisted selection with backcrossing. DNA markers also can help determine, regarding diversity questions, how populations of given species are distributed, how genetically distinct different populations are with respect to each other, and how much genetic variation is present in and among populations.

Many different techniques for obtaining DNA markers have been developed in recent years, which include:

### RFLP (Restriction Fragment Length Polymorphism) :

The RFLP technique is based on the fact that base substitution in a restriction endonuclease site or base insertions or deletions between sites can result in detectable differences in the length of DNA fragments when a DNA sample is digested with restriction enzymes. DNA from an organism is isolated and digested with suitable restriction enzymes and size fractionated by gel electrophoresis. The DNA fragments are then transferred to a membrane before being hybridized to radioisotope labeled probes. The RFLP method was the first and most widely used method in obtaining genetic markers and provides very reproducible results. In many species, however, such polymorphism occurs at a low frequency, and the RFLP method requires large amounts of DNA, which in turn requires that a large amount of the sample be collected.

### RAPD (Random Amplified Polymorphic DNA) :

PCR (Polymerase Chain Reaction) amplifies genomic DNA with a single random primer of about ten bases, and the amplified products are separated by agarose gel electrophoresis. It has the advantage of being cheap, simple and fast to perform, and does not require prior sequence information. However, this method can result in non-reproducible bands, faint bands, or spurious bands that cannot be scored confidently.

### DAF (DNA Amplification Fingerprinting) :

This technique is similar to RAPD, except that very short (e.g., five to eight nucleotide) random primers are used for the amplification of genomic DNA, and the PCR fragments are imaged by polyacrylamide gel electrophoresis followed by silver staining. This method provides more variation, making it possible to distinguish between closely related cultivars. However, this method also suffers from the reproducibility and non-genetic origin problems of RAPD.

### SSR (Simple Sequence Repeats) :

In animal and plant genomes, microsatellite regions are rich in tandem repeats of two or five nucleotides. Such sequences are named simple sequence repeats. Such repeats are associated with high levels of polymorphism, and are well suited for generating genetic markers. Primers are used to amplify the SSR regions and the resulting PCR fragments are resolved by polyacrylamide gel electrophoresis. This method provides the high polymorphism of genetic origin, requires very little DNA, and give reproducible and repetitive results. However, prior sequence information is needed on multiple microsatellite loci for a particular organism in order to utilize this method of obtaining DNA markers.

### VNTR (Variable Number Tandem Repeats) :

Repetitive sequences of 10-100 base pairs occurring in tandem arrays of up to 1,000 units distributed throughout the genome are known as minisatellites. Variations in the number of tandem repeats of minisatellite DNA regions have been used as molecular markers to detect high levels of polymorphism, even between closely related individuals among the population of a single species. Mutation rates at minisatellite loci have been estimated to be as high as 2x10³ per meiosis. VNTR is widely used in forensic investigations.

### AFLP (Amplified Fragment Length Polymorphism) :

AFLP is based on the selective PCR amplification of primer-ligated restriction fragments from a total digestion of genomic DNA. It requires only a small amount of genomic DNA, does not require prior sequence information, and has the ability to amplify sequences from a large number of restriction fragments. It provides high polymorphism of genetic origin and gives reproducible results.

In any of the above methods, after hybridization or PCR reactions and electrophoresis, data is obtained by a variety of methods including autoradiography, ethidium bromide staining, silver staining, and fluorescence detection. Fragment sizes are calculated or estimated from DNA size standards run either alongside the samples or mixed with the samples. A typical result comprises the detection or imaging of a pattern of DNA fragments of different sizes at different intensities or peaks, called a DNA "fingerprint." The uniqueness of any DNA fingerprint is dependent on many factors, mainly the source DNA and probe or primer combination, but also on reaction conditions. When reaction conditions are standardized, the DNA fingerprint becomes specific to the source DNA and probe or primer combination.

Improvements in technology have greatly increased the speed at which raw DNA marker data can be generated. In comparison data collection and analysis is less developed and is the main bottleneck in restricting the full exploitation of the potential associated with DNA fingerprinting methods. Manual interpretation of the raw data is tedious, time-consuming, and subjective, and the results from different batches even within the same laboratory are difficult to compare. Consequently, it is almost impossible to exchange and compare quantitative results from different laboratories. There thus exists in the art a need for a simple, standardized format for expressing DNA fingerprint data that will greatly facilitate the development of the entire field.

### SUMMARY OF THE INVENTION

The present invention provides a solution to the shortcomings of the prior art as discussed above.

In particular, the present invention provides a method for storing DNA fingerprint data, comprising the steps of:
(a) providing a DNA fingerprint comprising resolved labeled DNA markers and a DNA size standard that are labeled to emit a detectable signal (100);
(b) detecting the signals emitted from said labeled DNA markers and measuring the peak intensity and location of said signals (102); and
(c) determining the size of said labeled DNA markers by comparison with said DNA size standard (102), characterised in that the method further includes:
(d) classifying the peak intensity of said signals emitted from said labeled DNA markers according to a predetermined discrete intensity level scale (108);
(e) creating a data record, wherein the step of creating a data record comprises, for each labeled DNA marker, associating a value with the labeled DNA marker wherein the value corresponds to the classified peak intensity of the signal emitted from the said labeled DNA marker and entering the value into the data record at a predetermined location within the data record, wherein the location of each said value within the data record is a function of the size of the labeled DNA marker with which the value is associated (112); and
(f) storing said data record (114).

According to another aspect of the invention, a computer program product is provided, comprising a computer-readable medium having computer-executable code recorded thereon for storing DNA fingerprint data, characterised in that said computer-executable code comprising:
means for storing data corresponding to the peak intensity of the signal emitted by labeled DNA markers and the size of each DNA marker in a DNA fingerprint;
means for classifying the peak intensities of said signals emitted by labeled DNA markers according to a predetermined discrete intensity level scale;
means for creating a data record, wherein the step of creating a data record comprises: means for associating each labeled DNA marker with a value that corresponds to the classified peak intensity of the signal emitted from said labeled DNA marker and means for entering the values into the data record at predetermined locations within the data record, such that the location of each said value within the data record is a function of the size of the labeled DNA marker with which the value is associated; and
means for storing said data record in a computer-readable storage medium.

According to another aspect, the invention provides a computer system, comprising: a computer, and a computer-readable storage medium having a DNA fingerprint data record stored therein, wherein said DNA fingerprint was obtained from a genomic DNA source, characterised in that said data record comprises:
an information field including:
   (a) information identifying the genomic DNA from which the DNA fingerprint was obtained,
   (b) information identifying the type of DNA fingerprint,
   (c) information identifying the spacing between successive DNA markers,
   (d) information identifying the starting size of said DNA markers, and
   (e) information identifying the ending size of said DNA markers; and
a sequence field containing a sequence of transformed DNA marker signal peak intensity symbols, wherein
said computer is programmed to read said data record from said storage medium and convert said sequence of transformed DNA marker signal peak intensity symbols into a DNA fingerprint record unique to said genomic DNA source identified in said information field.

### BRIEF DESCRIPTION OF THE DRAWINGS

The invention will be described in detail with reference to the following drawings in which:
Fig. 1 is a flow diagram of a method of processing DNA marker data according to one preferred embodiment of the present invention;
Fig. 2 is a table showing a format for expressing peak intensity of detected DNA fragments according to one embodiment of the invention;
Figs. 3A and 3B are diagrams for explaining a method of binning DNA fragments according to the invention;
Fig. 4 is a flow diagram of a method of binning DNA fragments according to the invention;
Fig. 5 is an example of an Amplified-type signature record of a DNA fingerprint according to the present invention;
Figs. 6A and 6B are tables providing values for carrying out scoring of DNA fingerprints according to the signature records obtained by the present invention; and
Fig. 7 is a flow diagram of the scoring process according to one preferred embodiment of the present invention.

### DETAILED DESCRIPTION OF THE PREFERRED EMBODIMENTS

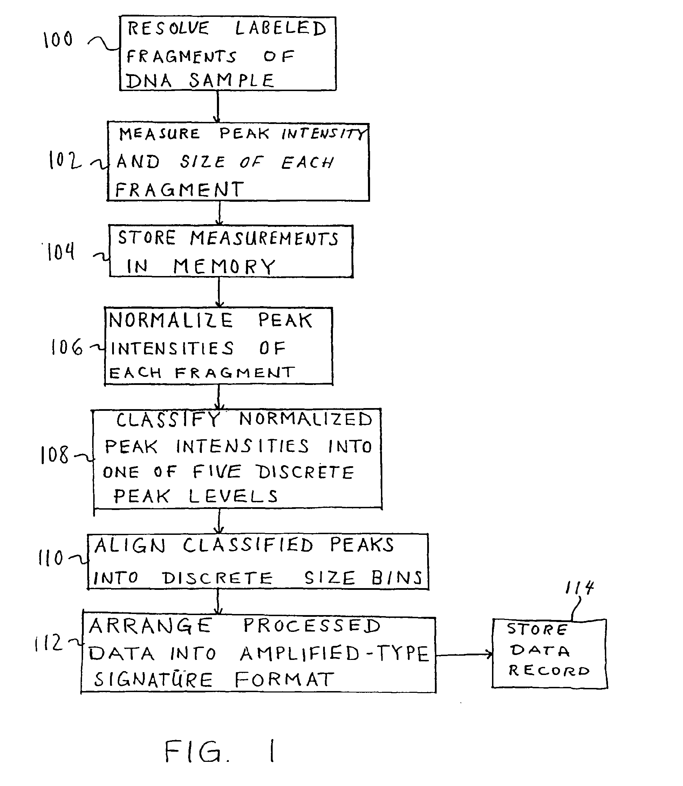

Referring to Fig. 1, one preferred method for DNA marker data analysis according to the present invention is illustrated. The analysis according to the invention preferably is carried out on a computer (such as a PC, minicomputer, mainframe, work station, server, etc.), using a software package performing the functions of the invention described in detail below.

At step 100, the labeled DNA fragments of a DNA sample that has been processed to obtain DNA markers according to any one of the abovementioned methods, are resolved, such as by running the sample through a gel electrophoresis. For example, using the AFLP technique, many small-sized PCR-generated fragments (typically in the range of 50-500 base pairs) are obtained, which comprise a DNA fingerprint. Depending upon whether radioisotope labeled primers or fluorescent labeled primers are used, different resolving processes are carried out. For radioisotope labeled primers, the samples are electrophoresed on a gel and the gel is exposed to X-ray film for several days. The exposed film is then manually interpreted.

For fluorescent labeled primers, the samples are run on an electrophoresis gel and fluorescent emission signals are detected in real time by a fluorescence sensor (such as a CCD camera or the like). The signals are digitized and inputted to a host computer for processing. Results are provided in tabular form. The use of fluorescent labeled primers is thus much faster than radioisotope primers and provides more reproducible results. An example of an automatic sequencer suitable for use in obtaining DNA marker data is the commercially available ABI 377 sequencer. Suitable DNA sequencers are available from a number of different manufacturers.

At step 102, the peak intensities of each fragment and the size of the fragment (given in bps or base pair length) are measured (such as by the fluorescence sensor coupled to a host processor or computer). The obtained measurements are stored in a memory storage medium (such as a solid state memory, hard drive, magnetic tape drive, etc.) at step 104.

Raw peak data can be obtained by inputting appropriate commands to the software running the DNA sequencer. For example, an "Export Lane-to-Raw" command in the software interfacing with the ABI 377 exports to memory a raw lane text file containing summarized peak data in columnar form, as illustrated below.

| | | | | | |
|---|---|---|---|---|---|
| G,1 | 35.29 | 51.49 | 151 | 544 | 683 |
| G,2 | 35.65 | 53.22 | 296 | 2046 | 690 |
| G,3 | 36.12 | 55.44 | 299 | 1834 | 699 |
| G,4 | 36.32 | 56.42 | 378 | 1529 | 703 |
| G,5 | 36.73 | 58.38 | 765 | 6140 | 711 |
| G,6 | 37.35 | 61.31 | 156 | 963 | 723 |
| G,7 | 37.72 | 63.01 | 171 | 979 | 730 |
| G,8 | 38.03 | 64.46 | 514 | 2982 | 736 |

The six columns represent, respectively, peak, time, size, height (intensity), area and point. The present invention makes use of only the size (i.e., number of base pairs in the fragment) and the height (i.e., the intensity).

At step 106, the peak intensities of the stored fragments are normalized. In this regard, the intensities may be normalized by average amplitude, wherein unity would represent the average height of the peaks over the entire size range of the waveform.

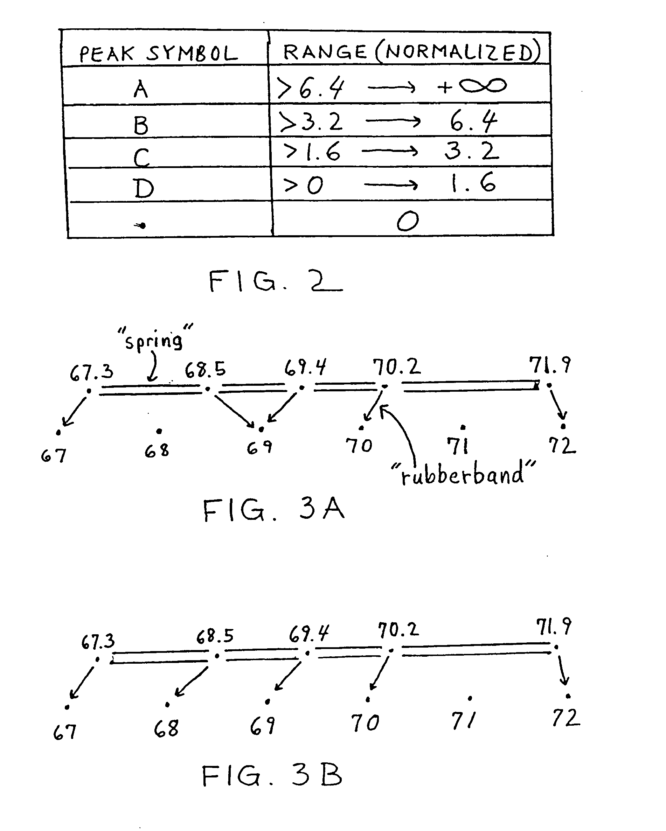

At step 108, the normalized peak intensities are classified or transformed into one of five discrete peak levels, as shown in Fig. 2. The empirical ranges shown in Fig. 2 were chosen after experimental adjustments, and were found by the inventors to work very well at producing consistent, reproducible signatures. According to one preferred embodiment of the invention, peaks are classified based on five discrete levels. This would allow adaptation to existing nucleotide sequence analysis software. However, peak intensities may be classified into any number of suitable levels.

The character set 'ABCD.' was chosen to represent the peak levels. The one-to-one correspondence between this character set and the universally used 'ATCGN' characters for representing DNA sequences enables adaptation of numerous sequence comparison software packages presently available on the market to the DNA fingerprint signature data of the present invention. In particular, such adaptation would involve simple adjustment of a software program's weight matrix to properly analyze the sequence as DNA marker peak data instead of DNA sequence data.

Next, at step 110, the stored DNA marker data are classified into discrete size (i.e., length) bins. Theoretically, an AFLP gel run should register peaks only at integral sizes (i.e., base pair lengths), because DNA fragments consist simply of multiple connected base pairs. However, because different base types are slightly different in size, peaks frequently are detected at fractional length sizes. In order to form the Amplified-type DNA marker signatures according to the invention, the detected peaks must be correlated to base pair positions, thus it is necessary to fit the experimental data into discrete sizes or "bins" each representing a base pair position or location starting from the beginning of a DNA fragment.

A typical method of binning is to form a ladder of the entire gel run, by lining up the gel lanes from the same run side-by-side so as to average out individual lane errors and to obtain a set of clear consensus rungs on the ladder into which the peaks are binned. However, this approach requires data from all lanes in the same gel run to be collected before the binning can be performed. This collective dependency exhibits an unacceptable deviation from the consistency and reproducibility requirements of the present invention, and thus a different method is used according to the invention, which allows an accurate and unbiased binning of peaks from a single lane, without the need to reference all other lanes in the run. Consequently, raw lane files of 2-3 Kb can be stored together with the generated DNA marker signature files according to the invention, which allows the signatures to be recalculated using later-developed algorithms.

A binning algorithm based on the borrowed concepts from physics of "spring" and "rubberband" energies is used according to the invention, since simple numeric rounding was found not to produce satisfactory results in binning a sequence of real-valued peaks. For example, as shown in Fig. 3A, for an experimentally obtained fragment sequence of sizes 67.3, 68.5, 69.4, 70.2 and 71.9, simple rounding would bin the sequence into 67, 69, 69, 70 and 72, obviously problematic since two peaks (68.5 and 69.4) would be binned into the same position although they are almost one whole position apart.

In order to arrive at a good fit, the relative distance between the peaks as well as their real positions is considered according to the invention. These two considerations can be combined mathematically into a single potential energy function, based on the physical models of a spring and a rubberband.

If one end of a compressible spring is fixed and the other end displaced by a distance x, the spring exerts a force acting in the direction against the displacement, and is expressed as F = -kx, where k is the spring constant, and the minus sign indicates that the force is opposite to the direction of the displacement x. Integrating the force function gives the potential energy of the spring, which is expressed as ½ kx², and which indicates that the potential energy is proportional to the square of the displacement.

The main difference between a spring and a rubberband is that a rubberband can only be pulled (expanded), and not pushed back (compressed) as can a spring. Functionally, this means that a rubberband is equivalent to a spring of zero length.

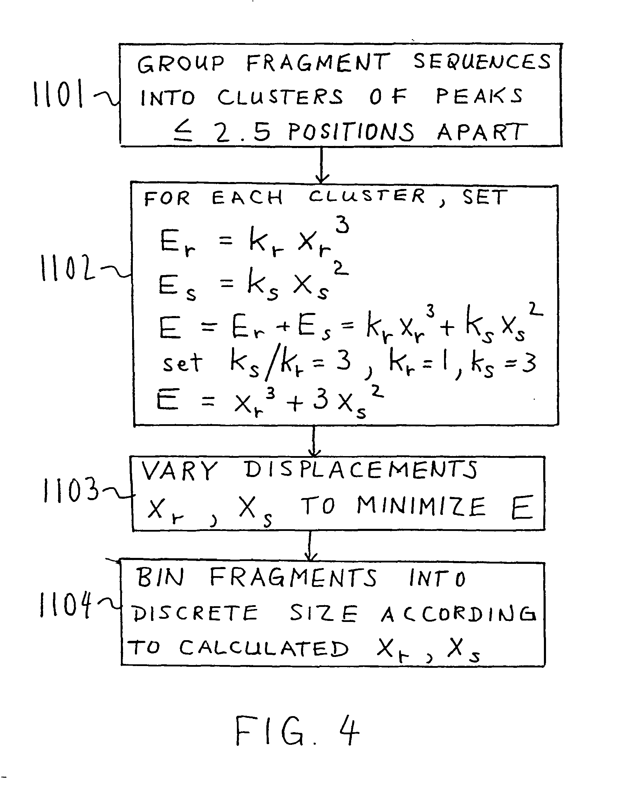

The Spring-and-Rubberband model is applied to the peak binning problem by assuming, as shown in Fig. 3A, that a spring maintains the distance between two adjacent peaks, and a rubberband pulls the real-valued peaks toward integral size points. A corollary of this model is that the binning should be localized within a close cluster of consecutive peaks, so as to allow sequence fragments to move together as a whole (and thus maintain their shape as much as possible), and to reflect the fact that relative distances between peaks become less significant as they become farther apart. Thus, according to a preferred embodiment of the binning process according to the invention, as shown in Fig. 4 at step 1101, fragment sequences are grouped into clusters of smaller fragment sequences consisting of adjacent peaks no farther than 2.5 positions apart. Optimal formulae for representing the energy of the springs and rubberbands for purposes of binning were experimentally determined as shown in step 1102 of Fig. 4, where Eᵣ is the rubberband energy, Eₛ is the spring energy, and E is the combined potential energy of the forces of the springs and rubberbands each acting on the peaks. In the equations, x is the displacement of the peaks and kᵣ and kₛ are the proportionality constants. Since the system works best when kₛ/kᵣ =3, kᵣ can be set to 1 and kₛ can be set to 3, such that E = xᵣ³ + 3xₛ².

At step 1103, the displacements xₛ and xᵣ are varied in a methodical fashion to achieve the lowest combined potential energy E, and thus the best fit to the discrete bin sizes. Once the lowest or minimum potential energy E is obtained, the peaks are binned according to the indicated displacements at step 1104. For example, as shown in Fig. 3B, the first "spring" is compressed from 1.2 units to one unit, while the second "spring" is stretched from 0.9 units to one unit (in contrast with the result of rounding as shown in Fig. 3A, where the first spring is stretched from 1.2 to 2 units, and the second spring is compressed from 0.9 to zero units). Similarly, the second and third "rubberbands" have respective displacements of 0.5 units and 0.4 units, as in Fig. 3A.

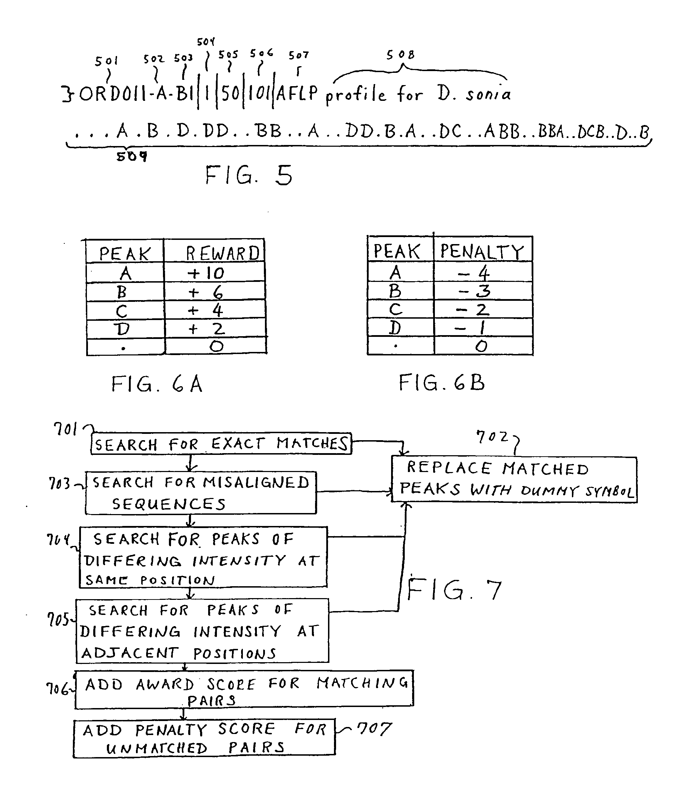

Once the normalized peaks are classified and the fragments binned into discrete sizes, at step 112 (Fig. 1) the DNA marker signature data is formatted into a standardized data entry record as shown in Fig. 5. The record has three distinct sections or fields: the first field is a unique accession code that functions as an identifier for a particular sample. The accession code contains information pertaining to the source DNA, the type of DNA marker generation technique, and the probe ID or primer combination. For example, as shown in Fig. 5, 501 designates a two-letter organism type (OR) and four-character alphanumeric organism ID, (D011) in this case an orchid cultivar. Reference numeral 502 designates a 1-character DNA marker technique, such as 'A' for AFLP, 'R' for RFLP, 'D' for RAPD, 'S' for SSR, and 'V' for VNTR (in the example, AFLP was used to generate the markers). Numeral 503 is a 1 or 2 primer pair code or probe code in the form of an alphabetic letter followed by a digit. In the case of primer combinations, an alphabet code indicates a primer in the 3' direction, and a digit code indicates a primer in the 5' direction. In the example, 'B1' indicates a predetermined primer combination for AFLP selective amplification.

The second field of the fingerprint signature according to the invention consists of other information, such as unit size (spacing), starting and ending positions, and a description of the sequence. Hence, numeral 504 indicates that the sequence spacing is 1 bp, numeral 505 indicates that the starting size for the fingerprint is 50, numeral 506 indicates that the ending size is 101, and 507 and 508 indicates an AFLP profile for D. *sonia.* The first line of the signature, containing the first and second fields, begins with a symbol such as a right brace (}); all subsequent lines not starting with '}' are interpreted as the signature sequence. Thus, signature sequence 509 in Fig. 5 gives the peak values for each fragment from 50 bp to 101 bp, in terms of the discrete levels A,B,C,D, and '.'. The data record so formed according to the invention is then stored at step 114 into a suitable data storage medium, such as a solid-state memory, magnetic or optical recording medium, floppy disk, hard disk, etc.

Among the many advantages of this new format are the immediately informative nature of the format, providing information on a peak's location and its relative intensity; results for different samples are aligned and thus easily compared; exchange of results among different laboratories is simple and straightforward (allowing simple text typing); fingerprints can be easily put into and retrieved from databases; and large-scale data analysis becomes possible. Thousands of fingerprints or more can be compared and analyzed, and markers linked to certain phenotype can be found through the use of computers, eliminating many man-hours of manual analysis.

To take full advantage of the novel data marker format, the signature must allow some degree of comparison to differentiate between similar and unrelated species. A scoring system is used which adapts the reward-penalty concept used in BLAST sequence comparisons, wherein a positive score is awarded for each matching character, and a negative score is given as a penalty for each mismatch.

Fig. 6A illustrates the peak reward scale, and Fig. 6B illustrates the peak penalty scale according to the invention. The score awarded for matching peaks is determined by their intensity. If both have positive peaks at a location, two sequences are said to match at that position. If the intensities are different (i.e., an 'A' matched with a 'C'), the lower of the peaks is used to obtain the score value. Alternatively, for every peak that exists on one sequence but is absent (i.e., zero) on the other, a penalty is subtracted from the total score, according to the value of the missing peak's intensity.

Thus, scoring AB..C.D against AB..C.D gives a total score of 22 (10+6+4+2), while scoring CB..A.D against CB....D, a -4 penalty is incurred for the missing 'A', giving a total score of 8 (4+6-4+2).

Fig. 7 illustrates a scoring method according to one embodiment of the invention. First, at step 701, two signature strings are compared with each other by searching for exact matches. Scoring is performed within a predetermined window, i.e., within a predetermined size range (such as from position 50 to position 350, for example). In the event that either of the sequences falls outside the boundaries of the window, the window is shortened to include only the range where both sequences have values.

As exactly matching peaks are found, at step 702 the matching characters are replaced in the string with a dummy symbol, to prevent peaks from being matched multiple times when additional searching is performed using modified search parameters (as described below). After all exactly matching peaks are found, at step 703 a search is performed on the remaining peaks to find peaks of the same intensity in a position immediately adjacent to the position of the peak under consideration. This step compensates for possibly misaligned sequences, where strings have matching peak intensities but are deviated from each other by one position, e.g., 'AB.CD.' with '.AB.CD'. In the event that matching peaks are found deviating by one position, the amount of the reward score is one half the points shown in Fig. 6A. Thus, scoring 'AB.CD.' against '.AB.CD' considering the one position deviation would result in a score of 11 (5+3+2+1), while interpreting the strings as having differing positive intensities at positions 2 and 5, with unmatched peaks at positions 1 and 6 and a C peak at position 4 deviating to a B peak at position 3, the resulting score would be reduced to 5 (-4+6+2+2-1). Matching peaks are again replaced with dummy characters at step 702, and processing advances to step 704, where a search is performed for peaks of differing intensity at the same position, and then step 705 where a search is performed for peaks of differing intensities at adjacent positions. Since all matched pairs of peaks have been progressively removed from the two strings being compared, the peaks remaining after step 705 represent mismatched peaks which will contribute to scoring penalties. At step 706 the award points are added for all previous matches, and at step 707 the (negative) penalty points are added for all mismatches, to arrive at a final score.

Negative scores are taken into account because sequences with mismatched peaks are considered even more different than sequences with no peaks. However, the use of negative scores makes the final score somewhat dependent upon the width of the scoring window, as the longer the sequences, the higher the probability of generating a higher overall score.

Because of this, a second scoring result is introduced which is expressed as a percentage value with a maximum of 100%. Within a designated scoring window, the percentage score of sequence B against sequence A is defined as the ratio of the score of B against A, to the highest possible score against A for the designated range (which would be attained if two identical A sequences were compared against each other).

While the score is a symmetric function in that score (A,B) = score (B,A), the percentage is an asymmetric function in that percent (A,B) = 100% x score (A,B)/score (A,A). The additional percentage metric thus is useful to indicate how good a match two sequences are within a given window. For example, considering a score between 'AB.CC.' and 'AB.CD.' (score = 22) compared with the same score for sequences in a wider window, such as 'ACD.A.CAB.CDC' and '.AB.C..BACD.C' (score = 22), the first pair of sequences is clearly a closer match than the second, which fact is reflected in the percentage scores when the second sequence of each pair is scored against the first of each pair (percentage = 93% versus 39%).

## Claims

1. A computer-based method for storing DNA fingerprint data, comprising the steps of:
(a) providing a DNA fingerprint comprising resolved labeled DNA markers and a DNA size standard that are labeled to emit a detectable signal (100);
(b) detecting the signals emitted from said labeled DNA markers and measuring the peak intensity and location of said signals (102); and
(c) determining the size of said labeled DNA markers by comparison with said DNA size standard (102), **characterised in that** the method further includes:
(d) classifying the peak intensity of said signals emitted from said labeled DNA markers according to a predetermined discrete intensity level scale (108);
(e) creating a data record, wherein the step of creating a data record comprises, for each labeled DNA marker, associating a value with the labeled DNA marker wherein the value corresponds to the classified peak intensity of the signal emitted from the said labeled DNA marker and entering the value into the data record at a predetermined location within the data record, wherein the location of each said value within the data record is a function of the size of the labeled DNA marker with which the value is associated (112); and
(f) storing said data record (114).

2. The method of claim 1, wherein said DNA fingerprint is an amplified fragment length polymorphism DNA fingerprint.

3. The method of claim 1, wherein said DNA fingerprint is a restriction fragment length polymorphism DNA fingerprint.

4. The method of claim 1, wherein said DNA fingerprint is a simple sequence repeats polymerase chain reaction DNA fingerprint.

5. The method of claim 1, wherein said DNA fingerprint is a variable number tandem repeats polymerase chain reaction DNA fingerprint.

6. The method of claim 1, further comprising the step of normalizing the measured peak intensities of said DNA marker signals (106) before (d).

7. The method of claim 1, wherein said discrete intensity level scale comprises at least five discrete peak levels.

8. The method of claim 1, further comprising the step of
aligning the sizes of said labeled DNA markers into corresponding ones of discrete size bins that have a value that correlates to a base pair number (110), wherein the step of aligning comprises the steps of:
grouping DNA marker sequences of said DNA markers into clusters, wherein adjacent peaks of each cluster are less than or equal to a predetermined number of discrete positions apart (1101);
assigning a potential energy value to each cluster, said potential energy value being proportional to the spacing between adjacent peaks of the cluster and to the amount of displacement required to bin the peaks into discrete size bins (1102);
varying the displacement of said peaks so that said potential energy value is minimized (1103); and
aligning said peaks into discrete size bins according to the displacement values that result in a minimized potential energy value (1104).

9. The method of claim 1, wherein said step of entering comprises the step of creating a data record having: an information field including
an identification of the source of the genomic DNA (501),
the type of DNA fingerprint (502),
the spacing between successive DNA markers (504),
the starting size of said DNA markers (505), and
the ending size of said DNA markers (506); and
a sequence field containing a sequence of said classified peak intensities (509).

10. The method of claim 9, further comprising the step of scoring a comparison between two data records, including the steps of assigning reward points to matches of peak intensities (706) and penalty points to mismatches of peak intensities (707), based on the relative magnitude of said peak intensities, comparing said two data records for identical matches at corresponding size positions (701), comparing said two data records for identical matches at adjacent size positions (703), comparing said two data records for non-identical matches at corresponding size positions (704), and comparing said two data records for non-identical matches at adjacent size positions (705), and totaling said reward points and penalty points according to found matches and remaining mismatches to obtain a score.

11. The method of claim 10, further comprising the step of obtaining a percentage metric for two compared data records, by obtaining a ratio of said score to a score obtained by matching one of said two data records to itself

12. A computer program product, comprising a computer-readable medium having computer-executable code recorded thereon for storing DNA fingerprint data, **characterised in that** said computer-executable code comprising:
means for storing data corresponding to the peak intensity of the signal emitted by labeled DNA markers and the size of each DNA marker in a DNA fingerprint;
means for classifying the peak intensities of said signals emitted by labeled DNA markers according to a predetermined discrete intensity level scale;
means for creating a data record, wherein the step of creating a data record comprises: means for associating each labeled DNA marker with a value that corresponds to the classified peak intensity of the signal emitted from said labeled DNA marker and means for entering the values into the data record at predetermined locations within the data record, such that the location of each said value within the data record is a function of the size of the labeled DNA marker with which the value is associated; and
means for storing said data record in a computer-readable storage medium.

13. The computer program product of claim 12, further comprising:
means for normalizing the measured peak intensities of said fragments before classifying said peak intensities according to said discrete intensity level scale.

14. The computer program product of claim 13, wherein said discrete intensity level scale comprises at least five discrete peak levels.

15. The computer program product of claim 12, further comprising means for aligning the sizes of said labeled DNA markers into corresponding ones of discrete size bins, wherein said means for aligning comprises:
means for grouping DNA marker sequences of said DNA markers into clusters, wherein adjacent peaks of each cluster are less than or equal to a predetermined number of discrete positions apart;
means for assigning a potential energy value to each cluster, said potential energy value being proportional to the spacing between adjacent peaks of the cluster and to the amount of displacement required to bin the peaks into discrete size bins;
means for varying the displacement of said peaks so that said potential energy value is minimized; and
means for aligning said peaks into discrete size bins according to the displacement values that result in a minimized potential energy value.

16. The computer program product of claim 12, wherein said means for entering comprises means for creating a data record having: an information field including
an identification of the source of the DNA fingerprint,
the type of DNA fingerprint,
the spacing between successive DNA markers,
the starting size of said DNA markers, and
the ending size of said DNA markers; and
a sequence field containing a sequence of said data record of classified peak intensities of DNA marker signals.

17. A computer system, comprising: a computer, and a computer-readable storage medium having a DNA fingerprint data record stored therein, wherein said DNA fingerprint was obtained from a genomic DNA source, **characterised in that** said data record comprises:
an information field including:
(a) information identifying the genomic DNA from which the DNA fingerprint was obtained,
(b) information identifying the type of DNA fingerprint,
(c) information identifying the spacing between successive DNA markers,
(d) information identifying the starting size of said DNA markers, and
(e) information identifying the ending size of said DNA markers; and
a sequence field containing a sequence of transformed DNA marker signal peak intensity symbols, wherein
said computer is programmed to read said data record from said storage medium and convert said sequence of transformed DNA marker signal peak intensity symbols into a DNA fingerprint record unique to said genomic DNA source identified in said information field.

## Patentansprüche

1. Computer-basiertes Verfahren zur Speicherung von DNA-Fingerprint-Daten, umfassen die Schritte:
(a) Bereitstellung eines DNA-Fingerprints, welcher aufgelöste markierte DNA-Marker und einen DNA-Größenstandard umfasst, die so markiert sind, dass sie ein detektierbares Signal emittieren (100);
(b) Detektieren der von den markierten DNA-Markern emittierten Signale und Messung der Peak-Intensität und der Lokalisierung der Signale (102); und
(c) Bestimmung der Größe der markierten DNA-Marker durch Vergleich mit dem DNA-Größenstandard (102), **dadurch** charakterisiert, dass das Verfahren darüber hinaus einschließt:
(d) Klassifizieren der Peak-Intensität der von den markierten DNA-Markern emittierten Signale entsprechend einer vorher festgelegten Skala diskreter Intensitäts-Levels, (108);
(e) Erzeugung eines Datenobjekts, wobei der Schritt der Erzeugung des Datenobjekts für jeden markierten DNA-Marker die Assoziation eines Wertes mit dem markierten DNA-Marker, wobei der Wert der klassifizierten Peak-Intensität des von dem markierten DNA-Marker emittierten Signals entspricht, und Eingeben des Werts in das Datenobjekt an einem vorher bestimmten Ort innerhalb des Datenobjekts umfasst, wobei die Lokalisierung jedes Werts innerhalb des Datenobjekts eine Funktion der Größe des markierten DNA-Markers ist, mit dem der Wert assoziiert ist (1 12); und
(f) Speicherung des Datenobjekts (114).

2. Verfahren nach Anspruch 1, wobei der DNA-Fingerprint ein AFLP (amplified fragment length polymorphism) DNA-Fingerprint ist.

3. Verfahren nach Anspruch 1, wobei der DNA-Fingerprint ein RFLP (restriction fragment length polymorphism) DNA-Fingerprint ist.

4. Verfahren nach Anspruch 1, wobei der DNA-Fingerprint ein SSR (simple sequence repeats) Polymerase-Kettenreaktion-DNA-Fingerprint ist.

5. Verfahren nach Anspruch 1, wobei der DNA-Fingerprint ein VNTR (variable number tandem repeats) Polymerase-Kettenreaktion-DNA-Fingerprint ist.

6. Verfahren nach Anspruch 1 , darüber hinaus umfassend den Schritt der Normalisierung der gemessenen Peak-Intensitäten der DNA-Markersignale (106) vor (d).

7. Verfahren nach Anspruch 1, wobei die Skala der diskreten Intensitäts-Levels mindestens fünf diskrete Peak-Levels umfasst.

8. Verfahren nach Anspruch 1 , darüber hinaus umfassend die Schritte
In-Verbindung-Bringen der Größen der markierten DNA-Marker mit entsprechenden diskreten Größen-Bereichen (discrete size bins), welche ein Wert aufweisen, der einer Basenpaaranzahl entspricht (1 10), wobei der Schritt des Verbindens die Schritte umfasst:
Gruppieren von DNA-Markersequenzen der DNA-Marker zu Clustern, wobei benachbarte Peaks jedes Clusters weniger oder gleich einer vorbestimmten Anzahl von diskreten auseinander liegenden Positionen sind (1101);
Zuordnung eines Potentialenergiewerts zu jedem Cluster, wobei der Potentialenergiewert proportional zum Abstand zwischen benachbarten Peaks des Clusters und zur Größe der Verschiebung ist, die erforderlich ist, um die Peaks in diskrete Größenbereiche einzuordnen (1102);
Variieren der Verschiebung der Peaks, so dass der Potentialenergiewert minimiert wird (1 103); und
In-Verbindung-Bringen der Peaks mit diskreten Größenbereichen entsprechend den Verschiebungswerten, die in einem minimierten Potentialenergiewert resultieren (1104).

9. Verfahren nach Anspruch 1 , wobei der Eingabeschritt den Schritt der Erzeugung eines Datenobjekts umfasst, welches ein Informationsfeld aufweist, das einschließt:
eine Identifikation der Quelle der genomischen DNA (501), den Typ des DNA-Fingerprints (502),
den Abstand zwischen aufeinander folgenden DNA-Markern (504)
die Ausgangsgröße der DNA-Marker (505), und
die Endgröße der DNA-Marker (506); und
ein Sequenzfeld, dass eine Sequenz klassifizierter Peakintensitäten enthält (509).

10. Verfahren nach Anspruch 9, darüber hinaus umfassend den Schritt des Scoring eines Vergleichs zwischen zwei Datenobjekten, einschließlich der Schritte Zuweisung von Pluspunkten für Übereinstimmungen von Peakintensitäten (706) und Strafpunkten für Nichtübereinstimmungen von Peakintensitäten (707), basierend auf der relativen Größe der Peakintensitäten, Vergleichen der beiden Datenobjekte im Hinblick auf identische Übereinstimmungen an entsprechenden Größenpositionen (701), Vergleich der zwei Datenobjekte hinsichtlich identischer Übereinstimmungen an benachbarten Größenpositionen (703), Vergleich der beiden Datenobjekte hinsichtlich nicht-identischer Übereinstimmungen an entsprechenden Größenpositionen (704), und Vergleichen der beiden Datenobjekte hinsichtlich nicht-identischer Übereinstimmungen an benachbarten Größenpositionen (705) und Addieren der Pluspunkte und Minuspunkte entsprechend den gefundenen Übereinstimmungen und verbleibenden Nichtübereinstimmungen zur Ermittlung eines Ergebnisses.

11. Verfahren nach Anspruch 1 0, darüber hinaus umfassend den Schritt der Ermittlung eines metrischen Prozentsatzes für zwei verglichene Datenobjekte, indem das Verhältnis des Scores zum Score ermittelt wird, der durch In-Übereinstimmung-Bringen eines der beiden Datenobjekte mit sich selbst ermittelt wird.

12. Computerprogrammprodukt, umfassend ein computerlesbares Medium mit einem darauf aufgezeichneten mit dem Computer ausführbaren Code zur Speicherung von DNA-Fingerprint-Daten, **dadurch** charakterisiert, dass der mit dem Computer ausführbare Code umfasst:
Mittel zur Speicherung von Daten, entsprechend der Peakintensität des von markierten DNA-Markern emittierten Signals und der Größe jedes DNA-Markers in einem DNA-Fingerprint;
Mittel zur Klassifizierung der Peakintensitäten der von markierten DNA-Markern emittierten Signale entsprechend einer vorherbestimmten Skala von diskreten Intensitätslevels;
Mittel zur Erzeugung eines Datenobjekts, wobei der Schritt der Erzeugung eines Datenobjekts umfasst: Mittel zur Assoziierung jedes markierten DNA-Markers mit einem Wert, der der kassifizierten Peak-Intensität des von dem markierten DNA-Marker emittierten Signals entspricht, und Mittel zur Eingabe der Werte in das Datenobjekt an vorherbestimmten Lokalisierungen innerhalb des Datenobjekts, so dass die Lokalisierung jedes Wertes innerhalb des Datenobjekts eine Funktion der Größe des markierten DNA-Markers ist, mit dem der Wert assoziiert ist; und
Mittel zum Speichern des Datenobjekts in einem computerlesbaren Speichermedium.

13. Computerprogrammprodukt nach Anspruch 1 2, darüber hinaus umfassend:
Mittel zum Normalisieren der gemessenen Peakintensitäten der Fragmente vor dem Klassifizieren der Peakintensitäten entsprechend der Skala diskreter Intensitötslevels.

14. Computerprogrammprodukt nach Anspruch 13, wobei die Skala diskreter Intensitätslevels mindestens fünf diskrete Peaklevels umfasst.

15. Computerprogrammprodukt nach Anspruch 12, darüber hinaus umfassend Mittel zum In-Verbindung-Bringen der Größen der markierten DNA-Marker mit entsprechenden diskreten Größenbereichen, wobei das Mittel zum Anpassen umfasst:
Mittel zum Gruppierung von DNA-Marker-Sequenzen der DNA-Marker zu Clustern, wobei benachbarte Peaks jedes Clusters weniger oder gleich einer vorherbestimmten Anzahl von diskreten auseinander liegenden Positionen sind;
Mittel zur Zuordnung eines Potentialengergiewerts zu jedem Cluster, wobei der Potentialenergiewert proportional zum Abstand zwischen benachbarten Peaks des Clusters und zur Verschiebung ist, die erforderlich ist, um die Peaks in diskrete Größenbereiche einzuordnen;
Mittel zum Variieren der Verschiebung der Peaks, so dass der Potentialenergiewert minimiert wird; und
Mittel zum In-Verbindung-Bringen der Peaks mit diskreten Größenbereichen entsprechend den Verschiebungswerten, die in einem minimierten Potentialenergiewert resultieren.

16. Computerprogrammprodukt nach Anspruch 12, wobei das Mittel zur Eingabe Mittel zur Erzeugung eines Datenobjekts umfassen, welche aufweisen: ein Informationsfeld, einschließlich
einer Identifikation der Quelle des DNA-Fingerprints,
des Typs des DNA-Fingerprints,
des Abstands zwischen aufeinander folgende DNA-Markern, der Ausgangsgröße der DNA-Marker, und
die Endgröße der DNA-Marker; und
ein Sequenzfeld, welches eine Sequenz des Datenobjekts der klassifizierten Peakintensitäten der DNA-Markersignale enthält.

17. Computersystem umfassend: einen Computer und ein Computerlesbares Speichermedium, welches gespeichert DNA-Fingerprint-Daten aufweist, wobei der DNA-Fingerprint aus einer genomischen DNA-Quelle erhalten wurde, **dadurch** charakterisiert, dass der Datensatz umfasst:
ein Informationsfeld, einschließlich:
(a) Information, die die genomische DNA identifiziert, von der der DNA-Fingerprint erhalten wurde,
(b) Information, die den Typ des DNA-Fingerprints identifiziert,
(c) Information, die den Abstand zwischen aufeinander folgenden DNA-Marker identifiziert, und
(d) Information, die die Ausgangsgröße der DNA-Marker identifiziert und
(e) Information, die die Endgröße der DNA-Marker identifiziert; und
ein Sequenzfeld, das eine Sequenz von transformierten DNA-Marker-Signalpeak-Intensitätssymbolen enthält, wobei
der Computer so programmiert ist, dass das Datenobjekt aus dem Speichermedium ausgelesen wird und die Sequenz transformierter DNA-Marker-Signalpeak-Intensitätssymbole in ein DNA-Fingerprint-Objekt konvertiert wird, das für die genomische DNA-Quelle einmalig ist, die im Informationsfeld identifiziert wird.

## Revendications

1. Procédé basé sur l'informatique pour le stockage de données d'empreintes d'ADN, comprenant les étapes constituées de :
(a) la fourniture d'une empreinte d'ADN comprenant des marqueurs d'ADN marqués résolus et un étalon de taille d'ADN, qui sont marqués pour émettre un signal détectable (100) ;
(b) la détection des signaux émis à partir desdits marqueurs d'ADN marqués, et la mesure de l'intensité du pic et la localisation desdits signaux (102) ; et
(c) la détermination de la taille desdits marqueurs d'ADN marqués par comparaison avec ledit étalon de taille d'ADN (102), **caractérisé en ce que** le procédé inclut en outre :
(d) la classification de l'intensité du pic desdits signaux émis à partir desdits marqueurs d'ADN marqués selon une échelle de niveau d'intensité discrète prédéterminée (108) ;
(e) la création d'un enregistrement de données, où l'étape de création d'un enregistrement de données comprend, pour chaque marqueur d'ADN marqué, l'association d'une valeur avec le marqueur d'ADN marqué où la valeur correspond à l'intensité de pic classée du signal émis à partir dudit marqueur d'ADN et l'entrée de la valeur dans l'enregistrement de données à une localisation prédéterminée à l'intérieur de l'enregistrement de données, où la localisation de chaque dite valeur à l'intérieur de l'enregistrement de données est une fonction de la taille du marqueur d'ADN marqué avec lequel la valeur est associée (112) ; et
(f) le stockage dudit enregistrement de données (114).

2. Procédé selon la revendication 1, dans lequel ladite empreinte d'ADN est une empreinte d'ADN de polymorphisme de longueur d'un fragment amplifié.

3. Procédé selon la revendication 1, dans lequel ladite empreinte d'ADN est une empreinte d'ADN de polymorphisme de longueur d'un fragment de restriction.

4. Procédé selon la revendication 1, dans lequel ladite empreinte d'ADN est une empreinte d'ADN de réaction de polymérisation en chaîne de répétitions d'une séquence simple.

5. Procédé selon la revendication 1, dans lequel ladite empreinte d'ADN est une empreinte d'ADN de réaction de polymérisation en chaîne de répétitions en tandem de nombre variable.

6. Procédé selon la revendication 1, comprenant en outre l'étape de normalisation des intensités de pic mesurées desdits signaux du marqueur d'ADN (106) avant (d).

7. Procédé selon la revendication 1, dans lequel ladite échelle de niveau d'intensité discrète comprend au moins cinq niveaux de pics discrets.

8. Procédé selon la revendication 1, comprenant en outre l'étape constituée de
l'alignement des tailles desdits marqueurs d'ADN marqués dans celles correspondantes de fichiers de taille discrets qui ont une valeur qui est en corrélation avec un nombre de paires de bases (110), dans lequel l'étape d'alignement comprend les étapes constituées par :
le groupement des séquences de marqueurs d'ADN desdits marqueurs d'ADN dans des blocs, dans lesquels les pics adjacents de chaque bloc sont espacés d'une longueur inférieure ou égale à un nombre prédéterminé de positions discrètes (1101) ;
l'assignation d'une valeur d'énergie potentielle à chaque bloc, ladite valeur d'énergie potentielle étant proportionnelle à l'espacement entre les pics adjacents du bloc et à la quantité de déplacement nécessaire pour ficher les pics en fichiers de taille discrets (1102) ;
la variation du déplacement desdits pics de sorte que ladite valeur d'énergie potentielle soit minimisée (1103) ; et
l'alignement desdits pics dans des fichiers de taille discrets selon les valeurs de déplacement qui aboutissent à une valeur d'énergie potentielle minimisée (1104).

9. Procédé selon la revendication 1, dans lequel ladite étape d'entrée comprend l'étape de création d'un enregistrement de données ayant un domaine d'information incluant
une identification de la source de l'ADN génomique (501),
le type d'empreinte d'ADN (502),
l'espacement entre les marqueurs d'ADN successifs (504),
la taille de départ desdits marqueurs d'ADN (505), et
la taille de fin desdits marqueurs d'ADN (506) ; et
un domaine de séquence contenant une séquence desdites intensités de pics classées (509).

10. Procédé selon la revendication 9, comprenant en outre l'étape de notation d'une comparaison entre deux enregistrement de données, incluant les étapes constituées de l'assignation de points de récompense aux appariements des intensités de pic (706) et de points de pénalité aux décalages des intensités de pic (707), sur la base de la valeur relative desdites intensités de pic, la comparaison desdits deux enregistrements de données pour des appariements identiques à des positions de taille correspondantes (701), la comparaison desdits deux enregistrements de données pour des appariements identiques à des positions de taille adjacentes (703), la comparaison desdits deux enregistrements de données pour des appariements non identiques à des positions de taille correspondantes (704), et la comparaison desdits deux enregistrements de données pour des appariements non identiques à des positions de taille adjacentes (705), et le calcul du total desdits points de récompense et points de pénalité selon les appariements trouvés et les décalages restants pour obtenir une note.

11. Procédé selon la revendication 10, comprenant en outre l'étape d'obtention d'un pourcentage métrique pour deux enregistrements de données comparés, par l'obtention d'un rapport de ladite note à une note obtenue par l'appariement de l'un desdits enregistrements de données à lui-même.

12. Produit de programme d'ordinateur, comprenant un support lisible par un ordinateur ayant un code exécutable par un ordinateur enregistré dessus pour le stockage de données d'empreintes d'ADN, **caractérisé en ce que** ledit code exécutable par un ordinateur comprend :
un moyen pour le stockage des données correspondant à l'intensité de pic du signal émis par les marqueurs d'ADN marqués et la taille de chaque marqueur d'ADN dans une empreinte d'ADN ;
un moyen pour la classification des intensités de pic desdits signaux émis par les marqueurs d'ADN marqués selon une échelle de niveau d'intensité discrète prédéterminée ;
un moyen pour la création d'un enregistrement de données, dans lequel l'étape de création d'un enregistrement de données comprend : un moyen pour l'association de chaque marqueur d'ADN marqué à une valeur qui correspond à l'intensité de pic classée du signal émis à partir dudit marqueur d'ADN marqué et un moyen pour entrer les valeurs dans l'enregistrement de données à des localisations prédéterminées à l'intérieur de l'enregistrement de données, de sorte que la localisation de chaque dite valeur à l'intérieur de l'enregistrement de données est une fonction de la taille du marqueur d'ADN marqué avec lequel la valeur est associée ; et
un moyen pour le stockage dudit enregistrement de données dans un support de stockage lisible par un ordinateur.

13. Produit de programme d'ordinateur selon la revendication 12, comprenant en outre :
un moyen pour la normalisation des intensités de pic mesurée desdits fragments avant la classification desdites intensités de pic selon une échelle de niveau d'intensité discrète.

14. Produit de programme informatique selon la revendication 13, dans lequel ladite échelle de niveau d'intensité discrète comprend au moins cinq niveaux de pic discrets.

15. Produit de programme informatique selon la revendication 12, comprenant en outre un moyen pour l'alignement des tailles desdits marqueurs d'ADN marqués dans celles correspondantes de fichiers de taille discrets, dans lequel ledit moyen pour l'alignement comprend :
un moyen pour le groupement des séquences marqueurs d'ADN desdits marqueurs d'ADN dans des blocs, dans lesquels les pics adjacents de chaque bloc sont espacés d'une longueur inférieure ou égale à un nombre prédéterminé de positions discrètes ;
un moyen pour l'assignation d'une valeur d'énergie potentielle à chaque bloc, ladite valeur d'énergie potentielle étant proportionnelle à l'espacement entre des pics adjacents du bloc et à la quantité de déplacement nécessaire pour ficher les pics en fichiers de taille discrets ;
un moyen pour faire varier le déplacement desdits pics de sorte que ladite valeur d'énergie potentielle soit minimisée ; et
un moyen pour l'alignement desdits pics en fichiers de taille discrets selon les valeurs de déplacement qui aboutissent à une valeur d'énergie potentielle minimisée.

16. Produit de programme d'ordinateur selon la revendication 12, dans lequel ledit moyen pour l'entrée comprend un moyen pour la création d'un enregistrement de données ayant un domaine d'information incluant
une identification de la source de l'empreinte d'ADN,
le type d'empreinte d'ADN,
l'espacement entre les marqueurs d'ADN successifs,
la taille de départ desdits marqueurs d'ADN, et
la taille de fin desdits marqueurs d'ADN ; et
un domaine de séquence contenant une séquence dudit enregistrement de données des intensités de pics classées des signaux de marqueurs d'ADN.

17. Système d'ordinateur, comprenant : un ordinateur et un support de stockage lisible par un ordinateur ayant un enregistrement de données d'empreinte d'ADN stocké dedans, dans lequel ladite empreinte d'ADN a été obtenue à partir d'une source d'ADN génomique, **caractérisée en ce que** ledit enregistrement de données comprend :
un domaine d'information incluant :
(a) une information d'identification de l'ADN génomique à partir de laquelle l'empreinte d'ADN a été obtenue,
(b) une information d'identification du type d'empreinte d'ADN,
(c) une information d'identification de l'espacement entre les marqueurs d'ADN successifs,
(d) une information d'identification de la taille de départ desdits marqueurs d'ADN, et
(e) une information d'identification de la taille de fin desdits marqueurs d'ADN ; et
un domaine de séquence contenant une séquence de symboles d'intensité de pic de signal de marqueur d'ADN transformés, dans lequel
ledit ordinateur est programmé pour lire ledit enregistrement de données à partir dudit support de stockage, et convertir ladite séquence des symboles d'intensité de pic de signal de marqueur d'ADN transformés en un enregistrement d'empreinte d'ADN unique à ladite source d'ADN génomique identifiée dans ledit domaine d'information.
